# EUROPEAN PATENT SPECIFICATION

(11) **EP 2 718 088 B1**
(45) Date of publication and mention of the grant of the patent: **13.04.2016**
(21) Application number: 12743644.2
(22) Date of filing: 21.05.2012
(51) Int. Cl.: B29C 49/00, B29C 49/22, B65D 81/24, B29C 45/18, B29C 45/16, B65D 81/26, B65D 81/30

(54) **METHOD OF INJECTION MOULDING OBJECTS AND APPARATUS FOR MANUFACTURING THE OBJECTS**
VERFAHREN ZUM SPRITZGIESSEN VON KÖRPERN UND VORRICHTUNG ZUR HERSTELLUNG DER KÖRPERN
PROCÉDÉ DE MOULAGE DES OBJETS CREUX PAR INJECTION ET DISPOSITIF POUR LEUR FABRICATION

(30) Priority: 18.05.2011 BE 201100303
(43) Date of publication of application: 16.04.2014
(73) Proprietor: Resilux, 9230 Wetteren (BE)
(72) Inventor: DIERICKX, William, B-9070 Destelbergen (BE); DE CUYPER, Dirk, B-9070 Destelbergen (BE)
(74) Representative: Petsis, Christos
(86) International application number: PCT/BE2012/000027
(87) International publication number: WO 2013/000044

(56) References cited:
- EP-A1- 2 311 624
- EP-A1- 2 518 138
- WO-A1-2009/070800
- WO-A1-2010/034776
- WO-A2-2006/012713
- WO-A2-2006/099700
- DE-A1- 19 860 797
- US-A1- 2005 181 155
- US-A1- 2006 019 045

## Description

### Field of the invention

This invention relates to a hollow mould form, in particular a preform intended for producing containers which is constructed as a multilayered structure comprising a double base layer. The multilayered structure is composed of a primary material consisting of a polymer, with the formation of a double primary base layer as embedding matrix which forms the external surface of the preform which delimit this, on the one hand, and of a secondary intermediate layer which is included in the abovementioned double primary base layer comprising a secondary material, on the other hand.

### Background of the invention

Preforms consisting of primary and secondary materials are known. Containers are usually expected to ensure that the features and properties of the products contained therein remain as stable as possible over time.

EP 0380215 discloses a preform exists whose core forms a so-called barrier layer which is intended to counteract the migration of undesirable gas particles through the preform wall. However, it is composed of a material which is expensive to produce which, in certain cases, is an unacceptable limitation.

It is also known to produce a barrier layer in solid nylon^{®}, but this causes a further problem due to the fact that this material is very hydrophilic, as a result of which moisture may enter the preform via the barrier layer at the location of the gate of the preform. Such materials have the drawback that they have a pronounced tendency to absorb moisture, and thus cause delamination when the preform is processed further to form a container. Thus, the secondary core layer loses its barrier properties. Due to the known secondary materials, such as nylon, being highly hydrophilic, this type of known preforms develops a problem of penetration of moisture via the base thereof, which has a detrimental effect thereon. WO 2006 099700 discloses a method and a device for producing preforms using a primary material and a secondary cold materiel. However, in certain cases, there is a need to provide an additional tertiary material with a specific additional function which consists of improving the mechanical and/or thermal and/or barrier properties of the primary base material and, if desired, also of the secondary material.

In this respect, this additional tertiary material has specific properties which, in certain cases, may differ quite significantly from the primary base materials and secondary barrier materials, respectively. As a result thereof, a problem may develop with regard to the introduction of this tertiary material into the hollow mould.

### Aim of the invention

This consists of providing a hollow mould of the abovementioned type with a controlled addition of tertiary material, but with an effective barrier layer in a wide range of applications, on the one hand, and at acceptable costs, on the other hand.

### Summary of the invention

The arm is reached by a method and a device in accordance with claims 1 and 21.

To this end, a preform is proposed according to the invention as defined in the main claim, which is remarkable in that the abovementioned secondary material consists of a carrier material in which a tertiary material is incorporated as initiator or activator material, respectively, with the formation of an internal composite barrier intermediate layer.

Hollow moulds may be made of so-called monomaterial, in particular PET. A further possible composition thereof is that of the so-called bi-component, consisting of PET as primary base material and an, if desired different, secondary barrier material, such as nylon, in which the secondary material, if desired, can also be made of PET, but with the addition of a tertiary material.

According to an advantageous embodiment of the invention, this tertiary material consists of a fluid. Due to the use of a fluid as tertiary barrier material, the barrier layer can be applied with a specific additional function which is provided by this tertiary material, resulting in an improvement of the mechanical and/or thermal and/or barrier properties.

According to a particularly advantageous embodiment of the invention, this fluid is a specific liquid. The fluid material has its liquid phase at least during, possibly after the production process, in which the abovementioned secondary layer is a barrier layer. Each layer is virtually continuous and uninterrupted. Thus, a tertiary material is introduced therein as a fluid which contributes to the formation of the barrier layer. This offers the advantage that it forms an effective barrier along the preform -and therefore also the container- if desired including its neck part. In this connection, the best tertiary materials are those which have a relatively low viscosity under normal operating conditions regarding temperature and pressure, referred to below as "cold liquids".

It is also possible to apply such a tertiary material more evenly. A significant advantage in this connection is the fact that smaller amounts of secondary material can be introduced, resulting in a lower cost price. In addition, a structure of better quality is achieved which leads to a better barrier.

This is directly connected to the behaviour of fluids which naturally have a tendency to disperse homogeneously in a certain volume, resulting in significantly less delamination between layers.

In addition, as a result of the invention, it is thus possible to work with lower relative amounts of secondary material, while at the same time maintaining the same barrier properties and without the abovementioned problem of delamination, since, according to the invention, said secondary intermediate layer takes up less than 5%, preferably less than 1%, of the total weight of the preform, while securing the same barrier behaviour.

According to a preferred embodiment of the invention, the secondary layer has a markedly low thickness which is preferably not greater than 0,05 mm, preferably still < 0,01 mm.

Using specific liquid fluids produces the best uniform fillings of the space between the primary and tertiary layer, as liquids are essentially non-compressible, which supports uniformity. This advantage is very important as the product which is contained in the container made from the preform will thus maintain its features in an excellent manner without being subject to degradation over time, as a result of undesirable interactions with the environment or even with the inside of the container.

According to a particular embodiment of the invention, water may be considered, more specifically aqueous materials. These are understood to include the liquids in which water is bound. In this case, the excellent lamination which can be achieved herewith as a result of fewer compatibility problems between the various layers is particularly advantageous.

According to a further preferred embodiment of the invention, the secondary material in question is viscous, such as oil, more specifically an oil-carrying material.

A further advantageous embodiment of the invention consists in that said secondary material is formed by an acrylate based on water and/or oil. This type of liquid barrier is particularly suitable for so-called bio applications, since acrylates have very good oxygen barrier properties. In this case, the oil-containing acrylates are ideal carriers for so-called active barriers.

Thanks to the lamination effect which occurs with liquids, a preform according to the invention offers particularly remarkable properties. Due to the limited thickness of the secondary layer which can thus be achieved, it becomes possible to process so-called nanocomposites for the secondary layer, in particular based on clay, in which case it is, more particularly, possible to use the specific properties thereof to their full extent as a result of the lamination. This is actually made possible as a result of the possibility of applying the secondary layer in a more uniform manner. The latter material has a particular property of offering excellent coating power, while achieving a particularly good coverage.

According to a still further embodiment of the invention, the secondary material consists of a coating, in particular a polyurethane coating which has the advantage of good performance at elevated temperatures.

According to a particularly preferred embodiment of the invention, said secondary material consists of a liquid polymer, in particular a so-called polymer bio-aggregate (PBA) which is described in more detail in BE 2004/0431. The yeast strains and yeast cells are able to withstand higher temperatures up to approx. 170°C, provided that the period in question is relatively short, typically in the order of 10⁻¹ sec. Further features in this respect are defined in the respective sections.

According to an additional embodiment of the invention, said preform has a five-layered structure, in which said secondary layer is provided on at least one contact side with the adjacent primary layer of an intermediate layer composed of a quaternary material which is also made up of a liquid.

It should be understood that said fluids may also be gasses or may also comprise so-called intermediary phases, such as pastes and other substances which have a normal liquid phase, such as adhesives and the like.

According to a particularly advantageous embodiment of the invention, said secondary or quaternary layer(s), respectively, is provided with an adhesive layer which has been treated with yeast cells, so that mechanical bonding can be achieved with the primary and tertiary layers.

In a further advantageous embodiment of the preform according to the invention, at least the externally directed tertiary layer comprises a predetermined amount of additives which are incorporated in the tertiary plastic material. Due to the presence of additives, any constituents migrating from outside the container to the interior thereof are chemically bonded and thus neutralised, so that these constituents cannot reach the product which is contained in the container. Conversely, the additives in the primary layer ensure that constituents which are detrimental to the product contained in the container are likewise bound, so that these internal constituents cannot cause degradation of the product which is contained in the container.

According to a specific embodiment of the preform according to the invention, the primary layer contains additives having a neutralizing effect on external radiation, in particular UV radiation, thus producing a light barrier, more particularly in the invisible range, but also in the visible. This offers an advantage for products, such as milk, which mainly degrade on account of the effect of light - in particular UV - specifically the vitamins which are present in milk.

According to a still further embodiment of the preform according to the invention, the primary layer comprises additives with a neutralizing action on undesirable gas formation, thus producing a so-called gas barrier. This is, inter alia, intended to prevent the formation of CO₂ or oxygen which results from the degradation of the filler of the container and which is contained in the container together with the filler, in particular in a space above the filling level of the container where no filler of the drink type is present. Thus, oxidation of a drink packaged in the container can be prevented.

In a yet still further embodiment of the preform according to the invention, the internally directed layer comprises additives having a neutralizing effect on detrimental reagents which originate from the container itself and which originate in the plastic base material, in particular PET, during the production of the preform in the injection-moulding machine, in particular acetaldehyde. Although this product is not poisonous, it may nevertheless produce a sweet flavour since this gas is readily soluble in water at room temperature. Due to this measure which consists of the use of additives as so-called chemical blockers, the migration of acetaldehyde constituents from the wall of the preform into the product contained therein is counteracted, as a result of which it is possible to prevent the flavour of said product from changing. It could be established that the abovementioned chemical blockers can best be incorporated in the preform wall via a liquid barrier layer according to the invention.

In a preferred embodiment of the preform according to the invention, said secondary layer is formed by a passive barrier, with the secondary layer being at least less or only slightly permeable, preferably impermeable to a substance which possibly migrates there through, such as oxygen, carbon dioxide, and the like. Due to the presence of such a barrier layer, said possibly migrating constituents are blocked and thus prevented from migrating through the wall of the container. The barrier action of the barrier layer is bidirectional insofar as the intrusion of undesirable constituents, such as oxygen, into the container is counteracted in case the container contains products which would oxidize or degrade, perish or suffer a loss in quality on account of the gaseous constituents. Conversely, said barrier layer also prevents possibly desired gaseous constituents in the product contained in the container from migrating through the wall thereof from the inside to the outside, such as water to which additional carbon dioxide has been added, or drinks, such as soft drinks or beer.

In an advantageous manner, the migration of particles is counteracted in both directions, i.e. from the inside to the outside and from the outside to the inside.

According to a further additional embodiment of the preform, said secondary material has a chemical affinity with said undesirable substances which is such that the secondary material will react with the latter and will thus retain the undesirable substances in the wall of the container itself, so that the undesirable constituents cannot escape further or penetrate into the inside of the container.

In a specific embodiment of the preform, the secondary layer contains further additives having the same function as migration stopper, both with regard to undesirable gas constituents and radiation.

The present invention also relates to a method for producing hollow moulds by means of injection-moulding which are made from at least two materials, including a base material and a barrier material, in which the base material is formed by a plastic. Technologies for injecting primary and secondary materials are known. However, in certain cases, there is a need to inject an additional tertiary material with a specific additional function which consists of improving the mechanical and/or thermal and/or barrier properties to the primary base material and, if desired, also to the secondary material. In this respect, this additional tertiary material has specific properties which, in certain cases, may differ greatly from the primary base and secondary barrier materials, respectively. This may give rise to problems when introducing this tertiary material into the hollow mould.

Thus, according to this invention, a method is proposed for producing hollow objects, in particular multi-layered plastic preforms, made from a primary base material and a secondary material as carrier material, in which the base material and, if desired, the secondary material are made of a plastic. In this case, both primary base and secondary carrier materials are supplied to the hollow mould cavity via primary and secondary supply ducts in an injection mould and a hot-runner plate, each of which ends in fitting heating elements and their respective primary base and secondary carrier materials lead to a common gate via which the primary base and secondary carrier materials are injected into the hollow mould cavity separately. First, the primary base material is injected at a relatively high temperature T and pressure p. This method is characterized by the fact that, in addition, a predetermined amount of tertiary material is supplied externally as activator material, independently from both other materials, and outside the hot-runner plate, to the secondary supply duct into which the tertiary supply duct ends at a predetermined distance from the abovementioned common gate, upstream thereof, under temperature and/or pressure conditions which are considerably lower than the primary and secondary injection temperature T and/or injection pressure p. Thus, the tertiary material is carried along with the secondary material in order to be injected together with the latter into the hollow mould cavity in a controlled manner to form the composite intermediate barrier layer.
Indeed, it could be established that the controlled introduction of tertiary material can best be achieved by the secondary material carrying this tertiary material along during the injection stage of the former, namely when injection-moulding hollow moulds.

According to a preferred embodiment of the method according to the invention, in particular for producing a preform in an injection mould as determined in one of the respective product definitions, a predetermined amount of primary and secondary plastic material is injected into the hollow mould cavity at a relatively high pressure p and temperature T. In this case, the primary base material is injected in an uninterrupted or continuous manner, while the barrier material is applied to the primary material during the injection phase thereof via a gate and is carried along to said mould cavity over a short distance.

According to a further preferred embodiment of the invention, the tertiary material, being a thermally sensitive material, is supplied cold via a thermally unloaded path, in particular under virtually normal conditions of room temperature and atmospheric pressure. Thus, liquid can be supplied cold as tertiary material, directly via the gate.

According to a still further preferred embodiment of the invention, work is carried out under the operating temperature condition with a lower limit at approx. 100°C, under pressure conditions p≥1 atm, in particular at normal pressure conditions of approx. 1 atm, preferably up to a temperature of 30 to 40°C.

According to a yet further preferred embodiment of the invention, the tertiary material is carried along with the secondary material into a central region which is situated in the centre of the double primary base layer, with the formation of an internal barrier layer, which is composed from the secondary material and the tertiary material which has been incorporated therein as activator or initiator material.

According to a particularly preferred embodiment of the invention, the abovementioned material is supplied by means of a so-called insert or torpedo which is to this end placed in a fitted manner in the secondary supply duct at the edge of the hot-runner system just upstream of the gate. In this case, a certain amount of primary material is first injected into the mould through the abovementioned gate, after which the secondary material is supplied separately from the primary material via the secondary duct. In this case, a cooperating blocking bar is moved from its current primary position in which the secondary duct is blocked, to a further position in which the secondary duct is open and allows barrier material through in a cyclic manner via a further gate. Via the latter, the secondary and the tertiary material which is introduced via the diverted duct is injected in order to be injected together into the hollow mould cavity in a controlled manner.

According to a yet further preferred embodiment of the invention, the barrier materials are applied uniformly using relatively small amounts of materials, more particularly tertiary material.

According to a yet more preferred embodiment of the invention, a predetermined amount of additives is supplied before to at least one of the abovementioned materials in order to neutralize undesirable influences, more particularly external radiation and/or substances, in particular electromagnetic radiation, more particularly UV radiation, IR radiation, γ radiation and/or also visible light, or reagents which have an adverse effect on a product to be preserved, in particular by the action of the abovementioned photo-initiators.

Finally, the present invention also relates to an injection-moulding device for producing hollow objects, in particular plastic multi-layered preforms, comprising an injection mould having a hot-runner plate and a couple of injection nozzles. Each of these is provided with a centrally arranged main supply duct, at the free end of which a gate is provided which can be closed by means of a blocking bar which moves therein and can be moved to and fro, in which a primary duct is provided for ensuring the plastic base material is supplied hot to the gate. This device is remarkable in that a separate secondary duct is further provided for separately supplying the secondary carrier material to the gate which is common. In this case, the secondary material can first be supplied hot and separately from the primary material via the secondary duct, and then, downstream thereof, and can be supplied cold from said further gate. Furthermore, for the separate supply of a predetermined amount of tertiary material as activator material independently from the secondary material, a tertiary supply duct is provided which is external to the hot-runner plate and extends outside thereof towards said secondary supply duct. The tertiary supply duct opens herein, in a further gate which is provided at a predetermined distance from said common gate, upstream thereof, under temperature and pressure conditions, respectively, which are considerably lower than the primary and secondary injection temperature T and/or injection pressure p. Thus, the tertiary material can be supplied from this further gate together with the secondary material to the common gate. This allows the latter materials to be jointly injectable into the mould cavity via control means, which have been suitably provided for this purpose, in a controlled manner in the hollow mould cavity together with the tertiary material which can be supplied via the diverted duct. These consist of the cooperating blocking bar which is alternately displaceable between a current primary position for the primary supply and a further position for the supply of the composite barrier material. In particular, the duct provided for this purpose comprises an insert with a gate for the supply of primary material, and furthermore also a gate for the supply of secondary and tertiary material.

According to a particular embodiment of the device according to the invention, in particular for producing a preform in an injection mould as defined in one of the respective product claims, a secondary supply unit which is independent of the hot primary supply unit is provided which is arranged externally on the primary supply unit. Via this, secondary material can be supplied from an independent supply unit via a secondary supply duct which is provided for this purpose. A tertiary supply duct ending therein is provided and is arranged externally on the secondary supply unit, at least in the thermally loaded part thereof which is situated upstream of the abovementioned further gate, via which tertiary material can be supplied cold from an independent supply unit via the further independent supply duct provided for this purpose.

The tertiary supply duct is connected to the secondary supply duct in the further gate which is in turn also connected to the primary supply duct in a common injection nozzle which ends directly in the mould for producing the preform or container, respectively, in the gate thereof.

According to a still further particular embodiment of the invention, the abovementioned connection is provided at a relatively short distance from the injection point with respect to the inlet of the primary supply duct in said injection nozzle.

According to a yet further particular embodiment of the invention, in the device, in particular for carrying out a method as defined in one of the method claims, the thermally loadable primary and secondary materials can each be supplied separately via their respective extruders which are subjected to a thermal load extending up to approx. 270°C and higher, and which are both bypassed via a bypass which is formed by the abovementioned tertiary supply duct which is virtually thermally unloaded. Thus, as a result of the invention, the tertiary fluid can be introduced directly via the gate, while the known conventional screw in the heating block only serves to inject the primary or secondary layers, respectively.

Furthermore, there is also still the aspect of the hypersensitivity of the tertiary materials, in which case it is possible to work with completely inert materials compared to aggressive or damaging liquid materials due to the fact that the secondary material would otherwise damage the components of the injection nozzle considerably, depending on the nature thereof which depends on the desired application. Thus, the durability of the so-called hot-runner can be improved.

Further particulars and features are further defined in the appended subclaims.

Further details and properties, in particular of the preform or container, respectively, produced by means of a method or device, respectively, as defined in the related claims, are described in more detail in the following description of some exemplary embodiments of the invention by means of the attached drawings by way of example, in which identical reference numerals refer to the same or similar elements.

### Brief description of the drawings

Fig. 1 shows a diagrammatic representation of an embodiment of a manufacturing device for a preform for container according to the invention.
Fig. 2 and 3 each show a diagrammatic representation of an essential partial view of the injection-moulding device from Fig. 1.
Figs. 4 to 6 show diagrammatic representations of respective variant embodiments of a composite barrier structure in a preform according to the invention.
Fig. 7 shows a detail view of a part of the preform illustrated in Fig. 1 according to the invention.
Figs. 8 to 10 show a diagrammatic representation of a cross section along the axis of respective variant embodiments of a preform according to the invention.
Figs. 11 and 12 show a mixed representation of a container according to the invention, partly in front view, partly in cross section, with an enlarged view of a wall detail thereof, respectively.
Figs. 13 to 16 each show a diagrammatic representation of the functional operation of an essential part of the preform and container, respectively, according to the invention.
Figs. 17 to 19 each show a diagrammatic representation of the functional operation of an essential part of the injection-moulding device according to the invention, viewed in partial views and in cross section, respectively.
Fig. 20 shows the different supply paths for the primary, secondary and tertiary materials to a common gate upstream of the mould in the injection-moulding device from Figs. 17 to 19.
Fig. 21 is a diagrammatic representation of a further embodiment of a manufacturing device of the preform according to the invention.

### Description

Generally, this relates to an injection-moulding system for hollow shapes, in particular plastic preforms, more particularly multi-layered preforms, and containers which are made from monomaterial, for example PET as A material, but also from a 2 combi component material in which PET is used as A material and, for example, nylon is used as B material, or also, for example, PET as A material and PET as B material, but injected via two ducts with an added C material, more particularly a fluid, liquid or gas.

PET as A material and B material, respectively, may differ from one another, insofar as one is of high quality, in particular B, and the other one is of lower quality, in particular A.

An example of tri-component material is PET as A, nylon as B material and a viscous liquid as C material, both hot and cold, which is co-injected.

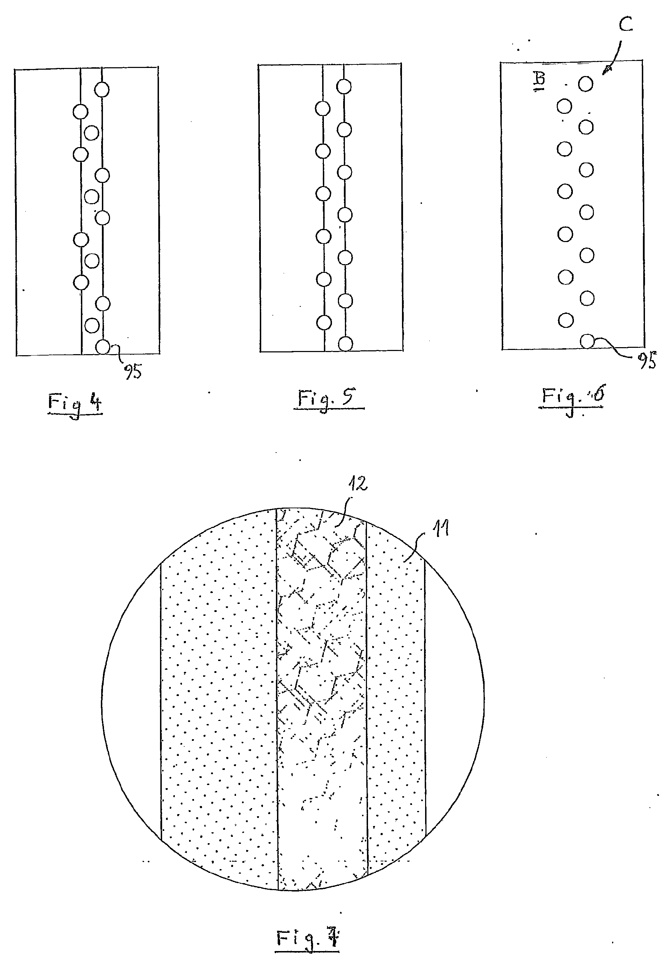

Figs. 4 to 6 show how C material is positioned in the wall of the preform 51, a fragment of which is illustrated in Fig. 7, in particular in a centre layer 12 or spread everywhere. If the tertiary C material is added in the one duct, it is spread everywhere 95, without a band as is illustrated in Fig. 6. If it is added to the other duct, it can normally be added to the B material, as is illustrated in Fig. 4. A third possibility is for the A material to be injected, i.e. while the C material is also distributed in said layer B material.

According to the known injection-moulding technique, liquids are added to PET material. If this goes through the entire extruder, then it is mixed and it is efficiently injected into a mould gate. However, with many liquids, a degradation takes place during this passage procedure.

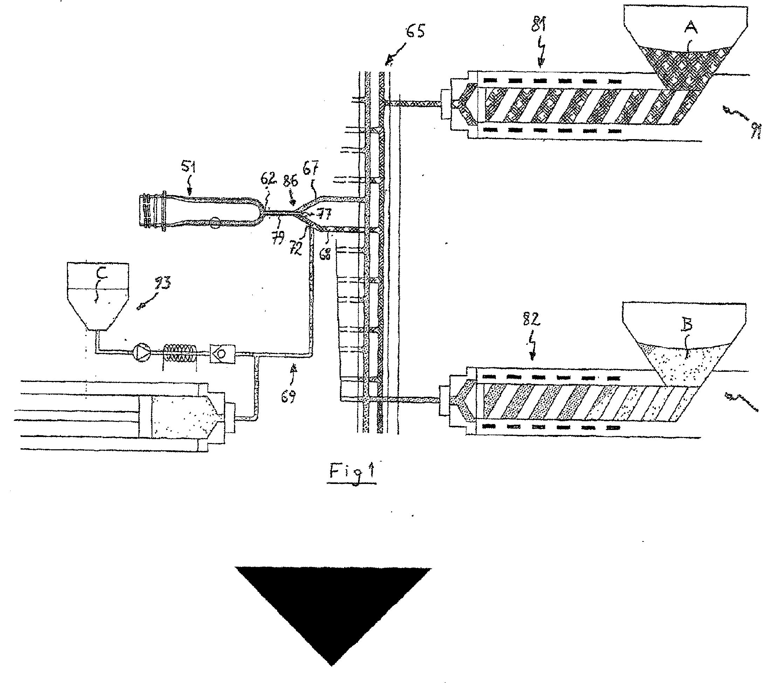

The advantage of this system according to the invention is that the C liquid, which is highly thermally sensitive, can be introduced externally via a bypass 69, in which case tertiary introduction can take place both cold and hot, as is illustrated in Fig. 1. Cold and hot means there are two aspects: there are liquids which can be introduced hot or cold, with it being possible to heat certain liquids to a certain temperature, into the injection nozzle part 86. C liquid can be introduced in the A material directly at the injection nozzle 86, or also unconventionally via the extruder 81, 82.

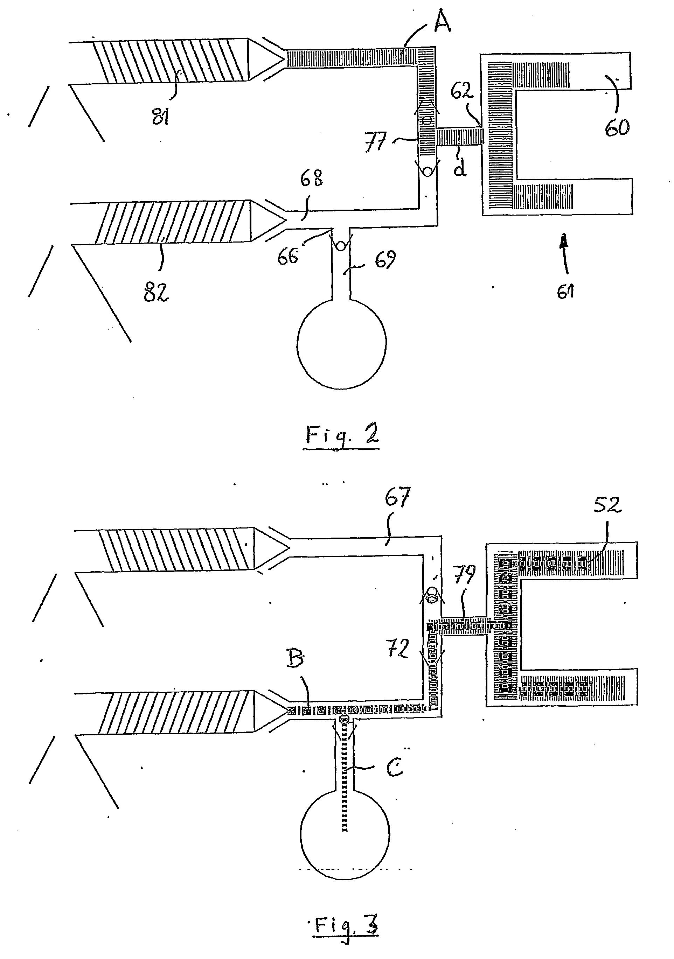

The main screw 81 is the conventional way, which is mainly critical in the case of bio barrier, for example, in which C liquid is not allowed to be subjected to excessive thermal loads in order to prevent microorganisms contained therein, such as spores, from dying before they are introduced into the mould 61. To this end, the liquid injection into the system according to the invention does not take place via the usual thermally loaded duct 67 and neither via the screws 81, 82, but by taking the C material precisely to a thermally less loaded part 69, in particular just before the product 51 is effectively produced at a short distance 79 from the gate 62. To this end, the liquid barrier 12 is brought about via a third duct 69, without the C material having to pass through the thermally highly loaded extruder 81 or 82, as is shown in more detail in Fig. 3.

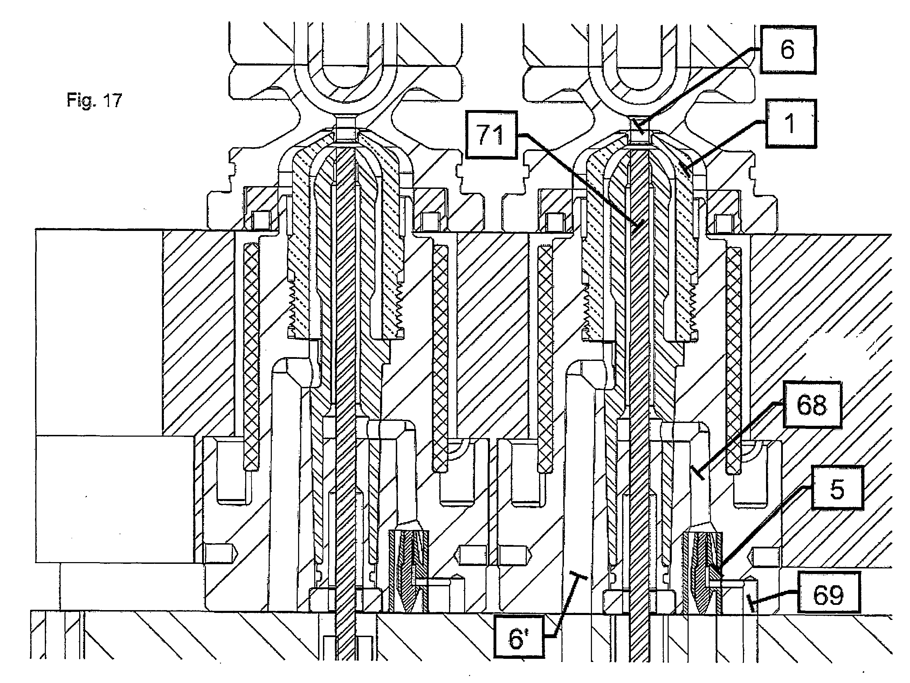

There is the small insert 5 which is situated in the duct 3, as is illustrated in Fig. 17. It is immaterial where the small insert 5 is situated, or whether it is at the front of the injection nozzle 86 or as far as the inlet of this material, in the A duct 67 or in the B duct 68.

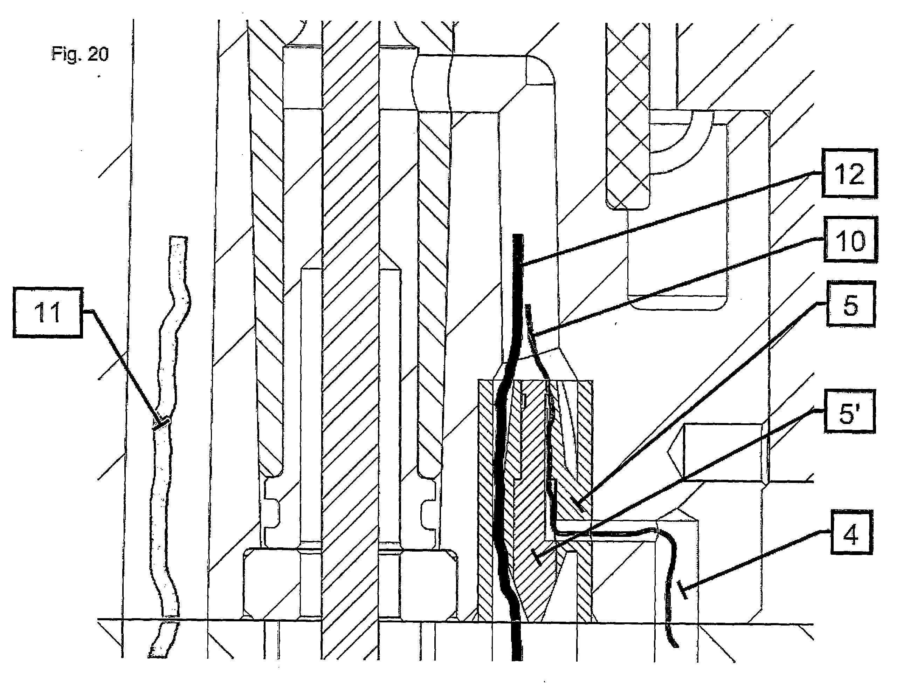

Fig. 20 shows the different supply paths of the primary, secondary and tertiary materials A, B and C, respectively, to the common gate 62, upstream of the mould 61, in the injection-moulding device according to A duct 67-2, B duct 68-3 and C duct 69-4, respectively. The small insert 5 may be either in this C duct, but it may also be in A duct 67 or in both. Said insert may be situated in any location in said duct. This has a practical effect if the insert is situated, for example, only in one duct or also in the other, or in both.

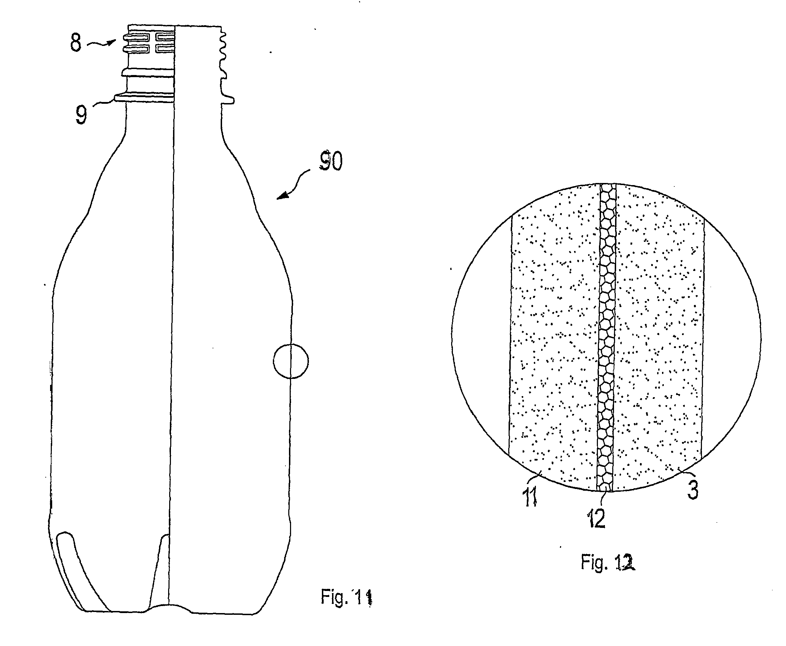

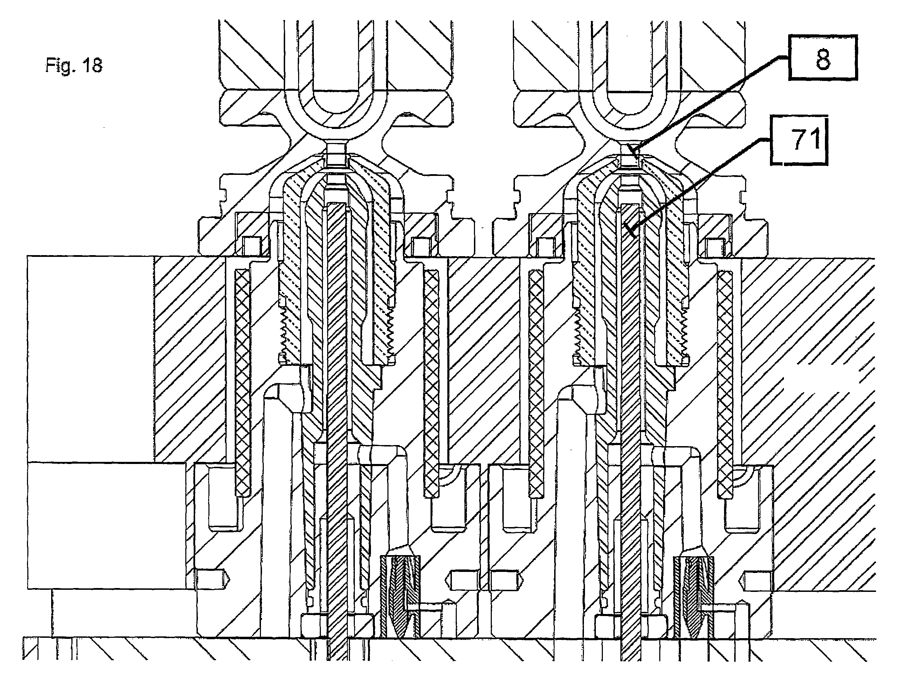

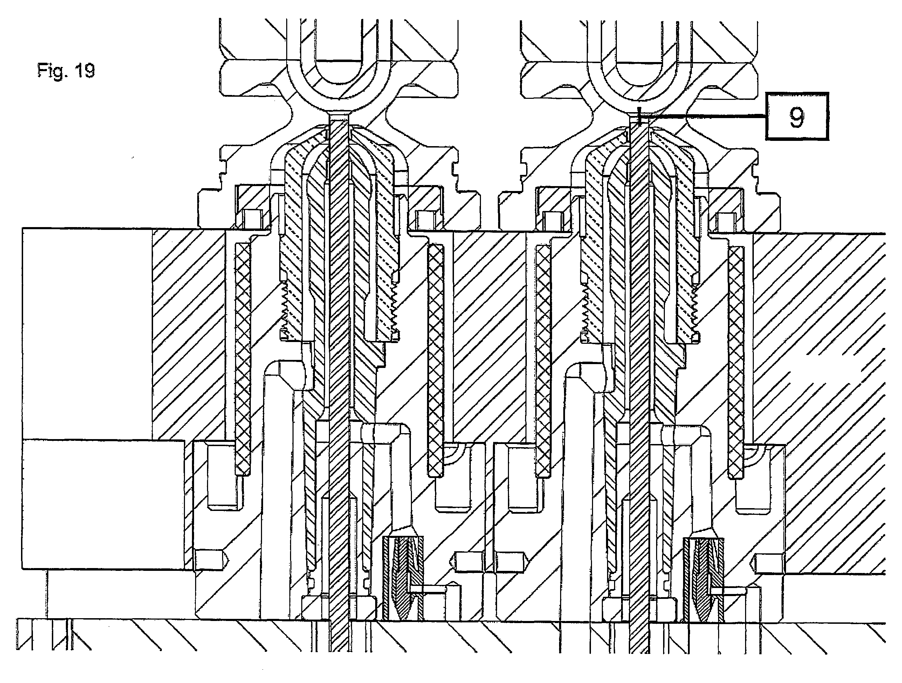

Figs. 17 to 19, in particular 20, show how the preform mould cavity 60 is filled. First, the A material is moulded on via primary supply duct 67, then the B material is added via secondary supply duct 68 which will flow exactly in the middle between the embedding matrix formed by A material, as is illustrated in Fig. 12. Then, the entire preform mould cavity 60 is filled by injection and a barrier layer 12 is created exactly in the middle. In this way, said layer 12 is constructed by means of cold-injection, one of the advantages of which is its suitability to the microorganisms, in which case this liquid may indeed be introduced by an injection of cold liquid, in the sense that this may reach 50° or 60°C in order to bring the viscosity to the desired level, for example pastes, understood to mean that it should rather be thermally less loaded conditions than the primary circuit 67.

In the example of the microorganisms, there is an A material, which is PET, where an intermediate layer 12 is laid comprising microorganisms. This is not possible if the microorganisms have to pass through the entire heated system 65. They may, if desired, keep this at 210°, but only for 30 seconds to 1 minute. Otherwise, all these microorganisms die. The aim is to develop the system in which the liquid containing the microorganisms is transformed into a barrier material. As a result of the invention, these microorganisms which are intended to be processed to form a barrier material can be introduced herein perfectly by allowing the injection to take place via a diversion 69 so they do not have to flow through the hot-runner 65.

A torpedo 5 can be placed in a fitted manner in front of this duct and it is possible to introduce both a liquid in the A material, e.g. microorganisms for the A material, and a second microorganism, but then for a CO₂ barrier for example in the B material, which are both introduced.

This system has three different injection gates 62, 72, 77 and it is possible to switch between these, for which reason there is a switching system with gates, in which case both A, B and AB can be supplied with the liquid for the product and thus a controlled introduction of the C material is achieved.

The nozzle 86 is diagrammatically represented in Figs. 2 and 3: first, there is a gate of A material, as is illustrated in Fig. 2, then, B material is added and a torpedo is placed with the introduction of C material, as is illustrated in Fig. 3.

Fig. 11 shows a container 50 made of plastic material, produced from the preform 51 using the so-called barrier technique with a multilayered structure comprising a barrier layer 12. This multilayered structure is shown in the preferred application of a preform as semifinished product in order to blow-form it to form a container. These are essentially containers in which beverages, foods, cosmetics and the like, both liquid and solid, or even gases, may be packaged and stored, such as bottles, pots, glass jars, jars, cans, jerry cans and the like.

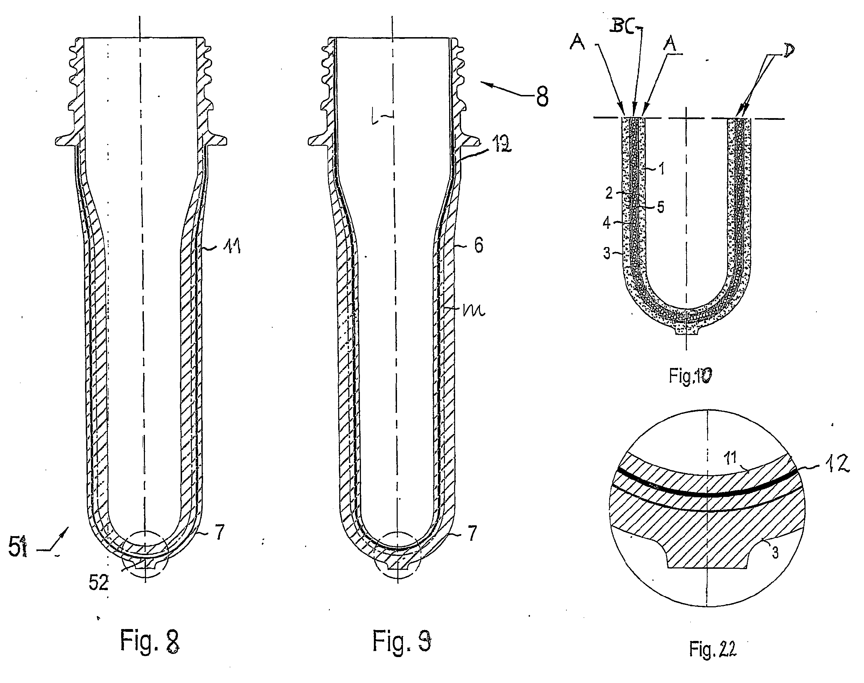

Figs. 8 to 10 show a container in its semifinished form in the shape of a preform 51 as a semifinished product essentially consisting of a neck part 8 which forms the pouring nozzle, an actual wall part 6 which is intended to be blown up to form a container 50 and a bottom part 7 which forms the base. The neck part 8 of the preform encloses a pouring opening 11' on the one side and then blends into the wall part 6 of the preform in a neck ring 9. At the bottom 7, the secondary layer has a deflection 47 to the bottom and extends further through the free end 49 thereof. This is illustrated in a more detailed view in Fig. 22. The latter embodiment is particularly suitable if said barrier layer 12 is directed more to the outside of the preform wall 6. This is the best embodiment for laminate applications which are typical for liquid barriers.

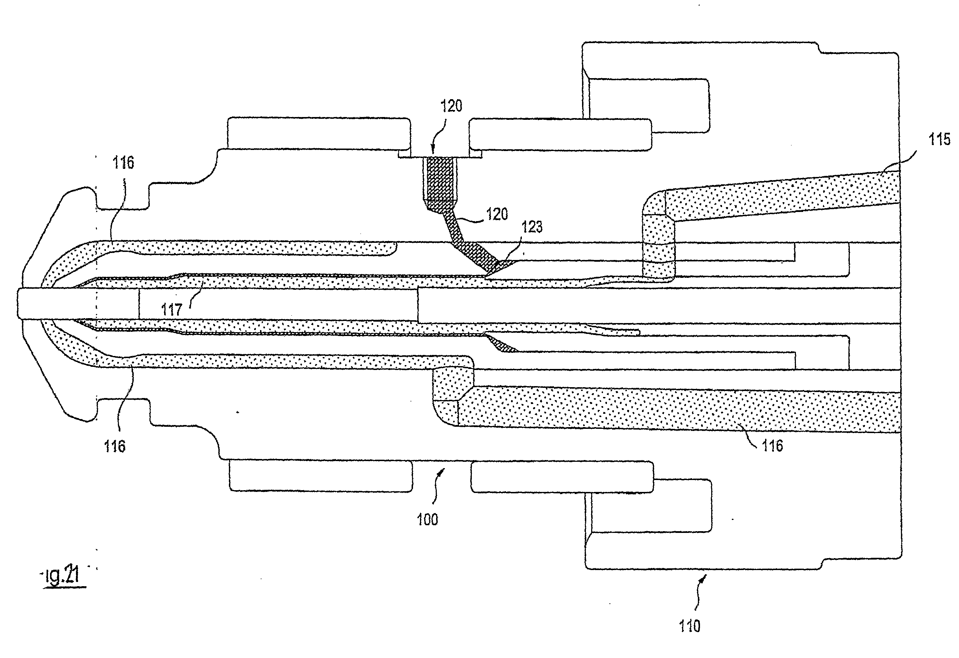

Fig. 21 furthermore diagrammatically shows an embodiment of a manufacturing device for producing a preform as described above, in which the primary and, if desired, tertiary plastic material is supplied in a known manner at a certain pressure p and temperature T via a primary supply unit 110, more particularly an injection-moulding device.

In this part 110 of the device, a relatively high temperature T prevails, typically in the order of magnitude of 240°C, which is such that the primary and, if desired, secondary plastic materials A, B which have been injected in a conventional manner are brought to a temperature which is higher than their melting temperature at atmospheric pressure. In conventional devices, the primary supply unit 110 is arranged at a certain distance from the bottom zone 7 of the injection mould intended for producing the preform, which is measured along the axis ℓ thereof. The supply to the injection mould is thus carried out by hot introduction from the processing unit 111 which has a screw 112 according to the well-known production process - as is described in EP-A-0686081, to which primary and, if desired, tertiary plastic material is supplied from a reservoir 113 via a supply duct system 114. The required base material for a specific preform is supplied via the respective supply duct 115 which is specifically intended for producing one preform 10.

Independent of the abovementioned hot introduction, a fluid is introduced in a characteristic manner at a temperature which is relatively colder, typically in the region of room temperature, compared to the abovementioned temperature T which prevails on the primary side. For this purpose, the tertiary material is introduced via a supply duct 122 which connects a fluid reservoir 121 to the gate 17 of the injection mould. Said fluid reservoir 121 is at room temperature and atmospheric pressure.

According to a variant as illustrated in Fig. 1, the tertiary material may also be supplied under pressure, e.g. via a pump 127 or an injection-moulding system, but without the screw. If desired, the tertiary supply temperature may be higher than room temperature, but significantly lower than the primary temperature T which prevails on the primary side.

The tertiary supply unit 120 is arranged on the exterior compared to the primary supply unit 110. This different arrangement with respect to one another makes said difference in operating temperature possible for both supply units tertiary 121 and both primary 113 and secondary, on the one hand, but also the use of a tertiary supply unit 120 which is significantly simpler with regard to construction, on the other hand.

Due to said cold introduction by means of said tertiary supply unit 120, fluids of the particular type which comprise the abovementioned PBAs can be used, having regard to the fact that these materials are very temperature-sensitive. Indeed, such PBAs cannot withstand temperatures which are necessary for the operation of the basic supply device 110.

Using the abovementioned separated supply unit 110, 120, a preform 51 is produced which has a three-layer structure, as is shown in Figs. 8 and 9. In this case, it should be noted that the tertiary supply unit 120 is arranged outside the primary 110 and secondary supply unit, respectively, in order to make the different operating temperatures possible, as described above.

In an embodiment of the manufacturing process of the preform, the primary layer 11 is produced by supplying primary plastic material via the respective supply ducts 114, while the intermediate layer 12 is produced by supplying the respective secondary or tertiary material, respectively, via the supply duct 122 provided for this purpose. If desired, secondary material is supplied via a supply gap 123 provided for this purpose. Fig. 21 shows a detailed representation of a mixing block 100. This diagrammatically illustrates how the secondary material is arranged at a relatively short distance d from the gate 62 upstream of the preform mould. Thus, the tertiary material is carried along with the secondary material by means of a lamination effect which is typical for liquid materials. In particular with the abovementioned bio-applications, in particular with the PBA's, the common supply distance 79 for primary, secondary and tertiary materials is kept as short as possible, so that the tertiary material, which is at a relatively colder temperature, is subjected to the significantly higher temperature of the primary and secondary materials coming from the heating block 110 for as short a period of time as possible.

Thus, the connection 37 is provided at a relatively short distance from the connecting point with respect to the inlet of the primary supply duct 115 in said injection nozzle 100 in order thus to limit the common path in which the secondary and tertiary material, on the one hand, and the primary material, on the other hand, are in contact with one another before the mould to a minimum time period. Thus, heating of the thermally fragile tertiary material by the primary material which is flowing along can be limited to a minimum which is a considerable advantage as, in some cases, excessive warming up is highly undesirable, particularly from certain levels, as is the case with the abovementioned PBA's.

Thus, the tertiary material is not supplied via the heating supply block, but outside of it, directly via the so-called injection nozzle 100.

Fig. 1 shows a variant of the device for producing a multi-layered preform. The primary plastic layers are produced by hot introduction of the primary materials A coming from the primary supply unit 110 via the supply duct system 114 provided for this purpose which has in each case one supply duct 115 for each preform to be produced, as described above. The tertiary material C is supplied via the tertiary supply unit 120, as described above, via the secondary supply ducts 122 provided for this purpose which, via a pressurized pump system 131, are subjected to the action of heating elements, such as resistors 132, for example. As a result thereof, the viscosity of the introduced fluid can be influenced by heating. Thus, the viscosity of the fluid can be reduced significantly, as with glues, for example, which are in particular intended to produce an intermediary glue layer 13, 14 in order to counteract delamination.

Fig. 8 shows the multilayered structure 11, 12, 13 of a preform 51, the base of which has a gate 57 via which primary A and barrier material BC can be injected into an injection mould 61 provided for this purpose, and the composite barrier material BC is supplied therewith. The external layer 11 of the preform is made of the primary material A, consisting of plastic, while the layer 12 forms an intermediate layer which is made of the composite barrier material BC and which forms a barrier layer.

Fig. 9 shows a variant in which the preform has a three-layered structure, comprising a primary layer 11 and a secondary intermediate layer 12, which are made of different plastics.

This barrier layer 12 can form both an active and a passive barrier in the sense that, with a passive barrier, the secondary material is impermeable or less permeable to a certain substance, such as O₂, CO₂, ...and the like, and blocks these. By contrast, with an active barrier, the barrier material reacts with a certain substance and in this way therefore retains damaging and/or undesirable substances in the wall, that is to say it will bind these chemically so that they cannot escape further or penetrate.

The barrier layer 12 forms at most 5%, but preferably not more than 1% of the total preform weight, depending on the application.

An example of the composition of the barrier layer 12 is oil or water, or preferably liquids carrying these substances. Another example of the composition of said secondary layer is PBAs, in which the yeast strains and yeast cells can withstand a higher temperature, up to approx. 170°C, provided that the respective period is relatively short.

Coatings, such as polyurethane coatings for example, also have the advantage that they perform well at relatively high temperatures.

Said primary and secondary plastic materials are advantageously polyethylene terephthalate (PET). However, said plastic materials can also be formed by polypropylene, polycarbonate and other polymers.

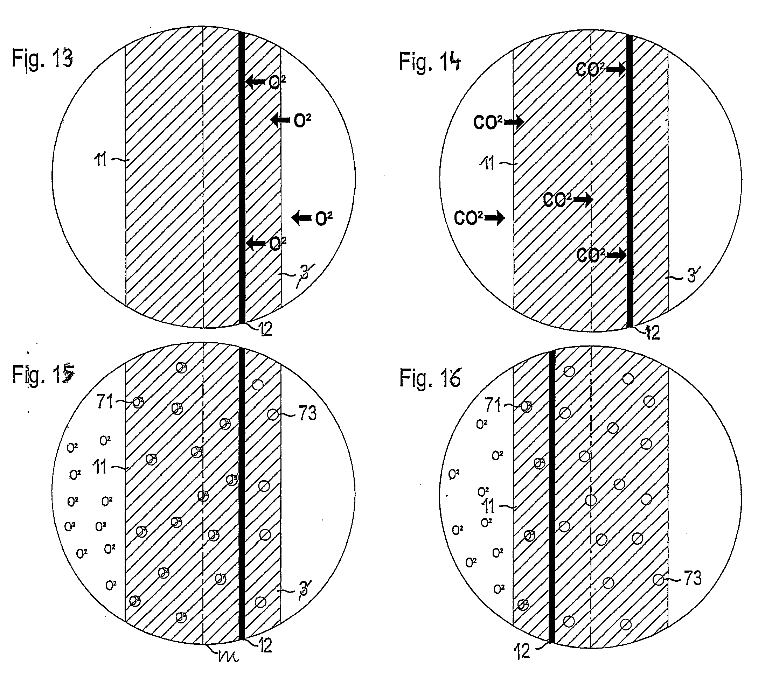

The primary and secondary materials may furthermore also be formed by plastic material to which additives 71, 73 have been added, as is illustrated in Figs. 15 and 16.

In addition, such containers 50 are completely recyclable. It is also possible for the primary material to be composed of a mixture of recycled materials and additives, if need be.

More particularly, additives 71 may be added to said primary plastic material, as is illustrated in Fig. 15, which binds the undesirable oxygen which migrates from outside the container to the inside, so that it cannot reach the product contained in the container.

This additive may also ensure that the oxygen which is contained together with the beverage in the container, in particular in a space on top of the filling level of the beverage, is bound, so that it is likewise prevented from causing oxidation.

A further additive may consist of colorants, by means of which a light barrier is produced as a barrier layer. Thus, for example, not only is UV radiation prevented from entering the container, but also visible light, since products, such as milk, and in particular the vitamins in the milk, degrade mainly due to the effects of light.

Another additive is a substance which binds acetaldehyde (AA). AA is a substance which develops in PET while producing the preform in the injection-moulding machine. Migration of AA from the wall of the bottle to the product in the bottle may cause a change in flavour, in particular with water containing carbon dioxide. CO₂ is a highly instable gas, as a result of which it will bind readily to other substances. This results in a sweet flavour, which causes an unpleasant taste, in particular in water, when it is drunk, and therefore this should be remedied as effectively as possible.

The above shows the effects of a suitable positioning of the intermediate layer 12 in the preform. After all, if the intermediate layer is located towards the outside of the preform, as illustrated in Fig. 8, this means that the PET layer containing additives is thicker there. If the additives 71 are situated in the primary PET layer 1, this makes it possible to bring more active constituents in contact with the product. Thus, more oxygen from the preform can be bound, as is illustrated in Fig. 13.

The tertiary material C is intended to form a barrier layer in order to block, e.g. oxygen, which would migrate through the wall of the container. Oxygen must be prevented from entering into the container if the container contains products which oxidize or degrade, perish or suffer a loss in quality on account of oxygen, such as fruit juice or milk, as is illustrated in Fig. 13.

This oxygen barrier layer is also important if the container contains water to which additional oxygen has been added. In this case, the layer prevents the oxygen from migrating through the wall from the inside to the outside, thus causing the quality of the water to deteriorate.

A barrier layer as illustrated in Fig. 14 is intended to block carbon dioxide which could migrate through the wall of the container from the inside to the outside. The loss of carbon dioxide in the container has to be prevented if the contents are, for example, a soft drink or beer, as the loss of CO₂ in this case results in a loss of quality of the beverage.

Furthermore, a gas barrier can have the same function of migration stopper for oxygen or carbon dioxide or UV radiation.

A further variant of preform 51 is partly illustrated in Fig. 10. This preform has five layers. The secondary layer 12 is provided on both sides with an intermediate layer 13, 14 which is made from a quaternary material QM which is formed by a fluid. Said fluid is advantageously formed by a glue which prevents the occurrence of delamination between the primary, secondary and tertiary layers of the preform. Very advantageously, each glue layer 4, 5 is treated with yeast cells.

A container 50 per se is illustrated in its finished form in Fig. 11, a detail at the location of the wall of which is illustrated in Fig. 12, which shows a wall of the type illustrated in Fig. 8 or 9 by way of example.

Said quaternary polymers may, for example, be polyolefin's, polyethylenes, PET, polypropylene, polyesters and other polymers.

### Empirical examples

1. A multi-layered preform was produced using PET as the primary material A, PET as the secondary material B and a highly hygroscopic liquid, coloured using 0.05% green colorant as tertiary material C. When opening the needles for the secondary material, the tertiary material was moulded onto the secondary material via a pump at a pressure of 220 bar. At a gate of 0.02 mm, an obstruction was caused by backflow and solidification of hot secondary material into the cold tertiary injection duct. This was prevented by reducing this gate to 0.01 mm. When, under these conditions, the temperature of the secondary material was increased to 340°C, a noticeable amount of liquid was added, which resulted in a visually green discolouration of the preform.
2. A multi-layered preform was produced using PET as the primary material A, PET as the secondary material B. Compressed air in the gaseous state was used as the tertiary material. Due to modifications in the nozzle, air bubbles in the preform were already detected at a temperature of the B material of 295°C and a pressure of 20 bar on the tertiary material. Upon cutting the preform, it could be seen that these air bubbles were located in the centre of the wall of the preform. Subsequently, a liquid colorant was added as the tertiary material. After the pressure on the tertiary material had been increased to 50 bar, a light green hue could be detected in the preforms. Upon cutting the preform, it could be seen that a clearly green centre layer had formed in the preform wall. This clearly shows that the tertiary material was added to the secondary material in small amounts.
3. A multi-layered preform was produced using PET as the primary material A, PET as the secondary material B and a 1:1 ratio of another hygroscopic liquid with green colorant. By modifying the liquid insert system and the associated torpedo, it was possible to freely control the starting time for moulding on the tertiary material and to achieve higher injection pressures, allowing improved control of the dosage of the tertiary material. In order to distinguish it from the clear A material and the green C material, the B material was coloured blue. In the preforms produced in this way, the three materials were clearly present.

It can be inferred from the tests described above that it is possible to add a liquid as a tertiary material to the secondary material in a multi-layered preform. By colouring both the secondary and the tertiary material, it was possible to visually ascertain that a preform produced in this manner consisted of two outer layers of primary material and a centre layer of secondary material into which the tertiary material is incorporated.

## Claims

1. Method for manufacturing hollow objects, in particular plastic multilayer parisons (51), which are manufactured in a hollow mold space (60) provided therefor from at least two materials, including a primary base material (A) and a secondary material (B) as a carrier material, wherein the base material (A), and possibly the secondary material (B), is formed by a plastic, and wherein both primary base and secondary carrier materials (A, resp. B) are fed to the hollow mold space (60) over primary and secondary feed channels (67, 68) in an injection mold (61) and a hotrunner plate (65), each of which (67, 68) run into adapted heating elements and their respective primary base and secondary carrier materials (A, resp. B) lead to a common extrusion connection sprue (62) through which the primary base and secondary carrier materials (A, B) are injected separately in the hollow molding space (60), first with the primary base material (A) which is injected under a relatively high temperature T and pressure p, **characterized in that** a predetermined amount of tertiary material (C) is further fed as activator material, independently from the other two materials (A, B), externally and outside of said hotrunner plate (65), to said secondary supply channel (68) into which a tertiary supply duct (69) runs (66) at a predetermined distance of said common extrusion connection (62), upstream thereof, under conditions of temperature and/or pressure which are significantly lower than for said primary and secondary injection temperature T and/or pressure p, so that said tertiary material (C) is entrained with said secondary material (B), in order to be injected together in the hollow space (60) in a controlled manner with the formation of an intermediate composite barrier layer (12); and **in that** said material (C) is supplied by means of an insert (5) or torpedo which is placed suitably therefor in the secondary feed channel at the edge of the hot runner system (65) just before the gate (62), wherein an amount of primary material (A) is first injected in said forming mold (60) through said extrusion connection sprue (62), whereupon said secondary material (B) is supplied individually with respect to said primary material (A) via the secondary channel (68), wherein a co-operating locking rod (71) is moved from its current primary position in which the secondary channel (68) is blocked, to a further position in which the secondary channel (68) is open and allows a cyclic passage of barrier material (B & C), through a further extrusion connection sprue (72) through which the secondary (B) and the tertiary material (C), which is incorporated via the bypass channel (69), are injected, in order to be injected together into the hollow mold space (60) in a controlled way.

2. Method according to claim 1, wherein a predetermined amount of primary, resp. secondary plastic material (A, B) is injected in the hollow molding space (60) under relatively high pressure p and temperature T, **characterized in that** the primary base material (A) is injected without interruption resp. continuously, while the barrier material (B and C) is supplied to said primary material (A) during the injection phase through a connection point (77), and is thus entrained over a short distance (79) to said mold space (60).

3. Method according to one of both preceding claims, for manufacturing a preform (51) into an injection mold according, wherein said hollow form is the preform intended for manufacturing containers (50), **characterized in that** said preform is made with a multi-layer structure comprising at least one first double base layer, which is composed of a primary material (A) consisting of a polymer, wherein a double primary base layer (11) is formed as embedding matrix, which constitutes the outer surface of the preform delimitating these, and which is further composed of a secondary intermediate layer (12) which is incorporated in said double primary base layer (11) comprising a secondary material (B), wherein said secondary material (B) consists of a carrier material in which a tertiary material (C) is incorporated as initiator or resp. activator material thereby forming an inner Intermediate composite barrier layer (12).

4. Method according to the preceding claim, **characterized in that** said primary material (A) is selected from a plastic polymer; and/or
**in that** said carrier material (B) is selected from a polymer, in particular a synthetic polymer, more particularly a biopolymer; and/or
**in that** said tertiary activator material (C) is selected from a fluid, particularly a liquid, in particular a viscous liquid, more particularly an oil-bearing fluid; or a water-containing, respectively aqueous liquid; or an acrylate, possibly oil or water based; or **in that** said fluid is formed by a gas.

5. Method according to one of the claims 3 or 4, **characterized in that** said secondary layer (12) is selected from a so-called intermediate phase, such as pastes, adhesives and other substances, which have a liquid phase under normal conditions of pressure and temperature, and which may possibly pass convertibly into a solid phase, in particular by curing; particularly wherein at least one additional intermediate layer (13,14) is provided at least at one contact side of said secondary layer (12) with the double primary adjacent layer (11), which (13, 14) is composed of a quaternary material (D).

6. Method according to one of the preceding claims, **characterized in that** said tertiary activator material (C) is selected from so-called photo-initiators (95); and/or
**in that** a predetermined amount of additives (41, 43), in particular dyes, is added to at least one of said materials (A,B,C,D) or layers (11, 12, 13, 14); particularly wherein said additives have a neutralizing action on external radiation and/or substances, in particular electro-magnetic radiation, more particularly UV-radiation, IR-radiation, γ radiation and/or visible light, if need be induction, and/or **in that** said additives have a neutralizing action on reagents having an adverse effect on a product to be preserved (58), and/or **in that** said additives (41) have a neutralizing action on undesired gas formation originating from the degradation of said product (58), and/or **in that** said additives have a neutralizing action on waste or degradation materials originating from the preform itself.

7. Method according to one of the claims 3 to 6, **characterized in that** the intermediate barrier layer (12) is made from a so-called polymer bio-aggregate which is composed by cells and/or cell products as activator material which is incorporated in said secondary polymer as a carrier material; particularly wherein said cells consist of so-called cysts and/or belong to the phase of the inactive or sleeping stages; or from yeasts, prokaryotes, in particular bacteria, and/or eukaryotes, notably of the protist type, fungi, and/or plants; resp.
**in that** said cell products consist of so-called metabolites, being the molecules which is synthesizable by organisms in a biochemical pathway.

8. Method according to the preceding claim, **characterized in that** said living organisms are incorporated into the polymer in a liquid phase during the injection molding at a temperature higher than 260°C; or when it is intended for manufacturing containers made from a polymer comprising living organisms, consisting of at least one layer (2), **in that** this layer is made of polyethylene terephtalate glycol comprising living organisms, wherein said polymer is PETG with a lower melting temperature than standard PET, in particular wherein said preform consists of at least three layers, wherein the layer of PETG is an intermediate layer that is comprised between the other two layers, more particularly wherein said living organisms are introducible in the PETG in a liquid phase during the injection molding process at a temperature higher than 200°C, even more particularly wherein injection molding is a co-injection molding process wherein the PETG can be injected at a colder temperature than the two layers on both sides of the preform;
in particular wherein said living organisms in the PETG belong to the species *Bacillus subtilis.*

9. Method according to one of the claims 1 or 2 to 8, **characterized in that** the tertiary material (C), which is a thermally sensitive material, is supplied cold via a path that is not thermally loaded (69), in particular at virtually normal conditions of room temperature and atmospheric pressure; and/or
in particular when depending on one of the claims 7 or 8, **in that** there is operated under the operating temperature Interval, the lower limit of which is set at substantially 100°C under pressure conditions p ≥ 1 atm, in particular under normal pressure conditions of about 1 atm, preferably at a temperature of up to 30° to 40°C; and/or
**in that** the tertiary material (C) is entrained with the secondary material (B) into a central region (52) that is comprised in the middle of said double primary base layer (11), with the formation of an inner barrier layer (12) which is composed of the secondary material (B) and the tertiary material (C) as activator or initiator material which is incorporated herein.

10. Method according to one of the preceding claims, **characterized in that** the barrier materials (B & C) are applied uniformly with very low amounts of material, more particularly tertiary material (C); and/or **in that** a pre-determined amount of additives is preliminarily fed to at least one of said materials with a neutralizing effect on undesirable influences, in particular on external radiation and/or substances, in particular electro-magnetic radiation, more particularly UV-radiation, IR-radiation, γ radiation and/or visible light, or on reagents which have an adverse effect on a product to be preserved.

11. Injection molding device for manufacturing hollow objects, in particular plastic multilayer preforms (51), comprising an injection mold (61), with a hotrunner plate (65) and a pair of injection molding nozzles (86), which are each provided with a substantially centrally arranged main supply duct (79) at the free end of which an extrusion connection sprue (62) is provided which is closable by means of a locking rod (71), which is movable therein and which is movable to and fro through a profiled inner part which is received in a support around which a heating element extends within which at least one primary channel (67) runs for the warm supply of plastic base material (A) to the sprue (62), wherein a separate secondary channel (68) is provided for the separate supply of the secondary carrier material (B) to the extrusion connection (62) which is common **characterized in that** said secondary material (B) is supplyable first warm and separately from the primary material (A), via the secondary channel (68), and subsequently downstream thereof, supplyable cold from further extrusion connection sprue (72), and **in that** a tertiary supply duct (69) is further provided for the separate supply of a predetermined amount of tertiary material as activator material (C) independently from the secondary material (B), which is external to the hotrunner plate (65) and which extends outwardly therefrom to said secondary supply duct (68) wherein the tertiary supply duct (69) runs into the further extrusion connection sprue (72) which is arranged at a predetermined distance (79) of the common extrusion connection (62), upstream thereof, under conditions of temperature resp. pressure which are significantly lower than said primary and secondary injection temperature T and/or pressure p, so that the tertiary material (C) is entrainable together with the secondary material (B) from the further extrusion connection sprue (72) to the common extrusion sprue (62), in order to be jointly injectable in the hollow mold space (60) in a controlled with the tertiary material (C), which is supplyable via the bypassed channel (69), via suitably provided control means for this purpose consisting of the co-operating locking rod (71) which is movable alternately between a current primary position for the primary supply, and a further position for the supply of the composite barrier material (B, C), wherein there is provided a secondary feed unit (92), which is independent from the hot primary feeding unit (91) and which is arranged externally to said primary feeding unit (91) through which secondary material (B) can be supplied from an independent power supply unit (99) via a secondary supply duct (68) provided therefor and **in that** a tertiary supply duct (69) running herein is provided which is arranged externally to said secondary feed unit (92) at least in the thermally loaded part thereof that is located upstream of said further extrusion connection (72), through which tertiary material (C) can be supplied cold from an independent power supply unit (93) via the further independent supply duct (69) provided therefor.

12. Device according to the preceding claim, in particular for manufacturing a preform in an injection mold, **characterized in that** the tertiary supply duct (69) is connected to the secondary supply duct (68) in the further extrusion connection sprue/ (72), which in turn is further connected to the primary supply duct (67) in a common injection nozzle (86), which runs directly into the mould (60) for the manufacture of the preform (51), resp. container (50), into the injection point (62) thereof.

13. Device according to the preceding claim, **characterized in that** said connection (77) is provided with the extrusion connection (62) at a relatively short distance (79) with regard to the input of the primary feed channel (11) in said injection molding nozzle (86).

## Patentansprüche

1. Verfahren zum Herstellen hohler Gegenstände, insbesondere von mehrschichtigen Vorformlingen (51) aus Kunststoff, welche in einer Hohlform (60) hergestellt werden, die dafür aus wenigstens zwei Materialien bereitgestellt wird, einschießlich einem primären Basismaterial (A) und einem sekundären Material (B) als Trägermaterial, wobei das Basismaterial (A) und möglicherweise das sekundäre Material (B) aus einem Kunststoff gebildet wird, und wobei so das primäre Basismaterial und das sekundäre Trägermaterial (A bzw. B) der Hohlform (60) über primäre und sekundäre Zuführkanäle (67, 68) in eine Spritzgussform (61) und eine Heißkanalplatte (65) zugeführt werden, wobei jeder derselben (67, 68) in angepasste Halselemente laufen und die jeweils primäres Basismaterial bzw. sekundäres Trägermaterial (A bzw. B) zu einem gemeinsamen Strangpress-Verbindungskanal (62) führen, durch welche das primäre Basismaterial und das sekundäre Trägermaterial (A, B) separat in die Hohlform (60) injiziert werden, zuerst das primäre Basismaterial (A), welches unter einer relativ hohen Temperatur T und relativ hohem Druck p injiziert wird, **dadurch gekennzeichnet, dass** ein vorbestimmter Betrag des tertiären Materials (C) zusätzlich als Aktivatormaterial unabhängig von den beiden anderen Materialien (A, B) extern und außerhalb der Heißkanalplatte (65) dem sekundären Zuführkanal (68) zugeführt wird, in welchem ein tertiärer Versorgungskanal (69) in einem vorbestimmten Abstand von der gemeinsamen Strangpress-Verbindung (62) verläuft (66), stromaufwärts davon, unter Temperatur-und/oder Druckbedingungen, welche deutlich niedriger sind als für die primäre und sekundäre Injektionstemperatur T und/oder Druck p, so dass das tertiäre Material (C) mit dem sekundären Material (B) mitgeführt wird, um zusammen in den Hohlraum (60) in einer gesteuerten Weise unter Bildung einer intermediären Verbund-Grenzschicht (12) injiziert zu werden; und dadurch, dass das Material (C) mittels eines Einsatzes (5) oder Torpedos zugeführt wird, welcher dafür in dem sekundären Zuführkanal an der Kante des Heißkanalsystems (65) kurz vor dem Gießkanal (62) in geeigneter Weise angeordnet ist, wobei eine Menge des primären Materials (A) zuerst durch den Strangpresse-Verbindungskanal (62) hindurch in die Hohlform (60) injiziert wird, woraufhin das sekundäre Material (B) individuell in Bezug zu dem primären Material (A) über den zweiten Kanal (8) zugeführt wird, wobei eine kooperierende Blockierstange (71) von ihrer gegenwärtigen primären Position, in welcher der sekundäre Kanal (68) blockiert ist, zu einer weiteren Position bewegt wird, in welcher der sekundäre Kanal (68) offen ist und einen zyklischen Durchgang des Barrierematerials (B & C) durch einen weiteren Strangpress-Verbindungskanal (72) erlaubt, durch welchen das sekundäre Material (B) und das tertiäre Material (C), welches durch den Umgehungskanal (69) eingeführt wird, injiziert werden, um zusammen in einer gesteuerten Weise in die Hohlform (60) injiziert zu werden.

2. Verfahren nach Anspruch 1, in welchem eine vorbestimmte Menge des primären bzw. sekundären Kunststoffmaterials (A, B) in die Spritzgussform (60) unter relativ hohem Druck p und relativ hoher Temperatur T injiziert wird, **dadurch gekennzeichnet, dass** das primäre Basismaterial (A) ohne Unterbrechung bzw. kontinuierlich injiziert wird, während das Trägermaterial (B und C) dem primären Material (A) während der Injektionsphase durch eine Verbindungsstelle (77) zugeführt wird und somit über eine kurze Strecke (79) zu der Spritzgussform (60) mitgenommen wird.

3. Verfahren nach einem oder beiden der vorstehenden Ansprüche, zum Herstellen einer Vorform (51) in einer Spritzgussform nach, wobei die Hohlform die zum Herstellen von Behältern (50) vorgesehene Vorform ist, **dadurch gekennzeichnet, dass** die Vorform mit einer Mehrschichtstruktur hergestellt wird, welche wenigstens eine erste Doppelbasisschicht umfasst, welche aus einem primären Material (A) zusammengesetzt ist, das aus einem Polymer besteht, wobei eine primäre Doppelbasisschicht (11) als einbettende Matrix gebildet wird, welche die äußere Oberfläche der Vorform bildet und diese begrenzt, und welche ferner zusammengesetzt ist aus einer sekundären Zwischenschicht (12), welche in der primären Doppelbasisschicht (11) eingefügt ist, mit einem sekundären Basismaterial (B), wobei das sekundäre Basismaterial (B) aus einem Trägermaterial besteht, in welchem ein tertiäres Material (C) als Initiator-bzw. Aktivatormaterial eingefügt ist, wodurch eine innere zusammengesetzte Zwischen-Barrierenschicht (12) gebildet wird.

4. Verfahren nach dem vorstehenden Anspruch, **dadurch gekennzeichnet, dass** das primäre Material (A) ausgebildet wird aus einem Kunststoffpolymer; und/oder dadurch, dass das Trägermaterial (B) ausgewählt wird aus einem Polymer, insbesondere einem synthetischen Polymer, ganz besonders einem Biopolymer; und/oder dadurch, dass das tertiäre Aktivatormaterial (C) ausgewählt wird aus einem Fluid, insbesondere einer Flüssigkeit, insbesondere einer viskosen Flüssigkeit, ganz bevorzugt einem ölhaltigen Fluid; oder einer wasserhaltigen bzw. wässerigen Flüssigkeit; oder einem Acrylat, möglicherweise Öl-oder Wasser basiert; oder dadurch, dass das Fluid durch ein Gas gebildet wird.

5. Verfahren nach einem der Ansprüche 3 oder 4, **dadurch gekennzeichnet, dass** die sekundäre Schicht (12) ausgewählt wird aus einer sogenannten Zwischenphase, wie Pasten, Haftmittel und andere Substanzen, welche eine flüssige Phase unter normalen Druck- und Temperaturbedingen haben und welche möglicherweise in eine feste Phase übergehen können, insbesondere durch Härten; insbesondere wobei wenigstens eine zusätzliche Zwischenschicht (13, 14) wenigsten an einer Kontaktseite der sekundären Schicht (12) mit der primären Doppelhaftschicht (11) vorgesehen wird, welche (13, 14) aus einem quartären Material (D) zusammengesetzt ist.

6. Verfahren nach einem der vorhergehenden Ansprüche, **dadurch gekennzeichnet, dass** das tertiäre Aktivatormaterial (C) aus sogenannten Fotoinitiatoren (95) ausgewählt wird; und/oder
dadurch, dass eine vorbestimmte Menge von Zusatzstoffen (41, 43), insbesondere Farben, wenigstens einem der Materialien (A, B, C, D) oder Schichten (11, 12, 13, 14) hinzugeführt wird; insbesondere wobei die Zusatzstoffe eine neutralisierende Wirkung gegenüber einer äußeren Strahlung und/oder Substanzen haben, insbesondere eine elektromagnetische Strahlung, insbesondere eine UV-Strahlung, IR-Strahlung, -Strahlung und/oder sichtbares Licht, bedarfsweise Induktion und/oder dadurch, dass die Zusatzstoffe eine neutralisierende Wirkung auf Reaktionsstoffe haben, die einen ungünstigen Einfluss auf ein zu konservierendes Produkt (58) haben und/oder, dass die Zusatzstoffe (41) eine neutralisierende Wirkung auf eine unerwünschte Gasbildung haben, die aus der Zersetzung des Produkts (58) herrührt, und/oder dadurch, dass die Zusatzstoffe eine neutralisierende Wirkung auf Abfall- oder Zersetzungsmaterialien haben, die aus der Vorform selbst herrühren.

7. Verfahren nach einem der Ansprüche 3 bis 6, **dadurch gekennzeichnet, dass** die Zwischenbarriereschicht (12) aus einem sogenannten Polymer-Bioaggregat hergestellt wird, welches durch Zellen und/oder Zellprodukte als Aktivatormaterial zusammengesetzt ist, welches in dem zweiten Polymer als Trägermaterial eingefügt ist; insbesondere wobei die Zellen aus sogenannten Bläschen bestehen und/oder zu der Phase der inaktiven oder schlafenden Stufen gehören; oder aus Hefen, Prokaryoten, insbesondere Bakterien und/oder Eukaryoten, nämlich des Protist-Typs, Hefen oder Pflanzen; respektive,
dadurch, dass Zellprodukte aus sogenannten Metaboliten bestehen, welche die Moleküle sind, welche durch Organismen in einem biochemischen Verlauf synthetisierbar sind.

8. Verfahren nach dem vorgehenden Anspruch, **dadurch gekennzeichnet, dass** die lebenden Organismen während des Spritzgussvorgangs bei einer höheren Temperatur als 260° C in das Polymer eingefügt werden; oder, wenn die Herstellung von Behältern aus einem Polymer vorgesehen ist, das lebende Organismen umfasst, bestehend aus wenigstens einer Schicht (2), dadurch, dass diese Schicht aus einem Polyethylenterephtalatglykol mit lebenden Organismen hergestellt ist, wobei das Polymer ein PETG mit einer niedrigeren Schmelztemperatur als ein Standard PET ist, insbesondere wobei die Vorform aus wenigstens drei Schichten besteht, wobei die Schicht aus PETG eine Zwischenschicht ist, die zwischen den anderen beiden Schichten eingefügt ist, insbesondere wobei lebende Organismen in das PETG in einer flüssigen Phase während des Einspritzvorgangs bei einer Temperaturhöhe von 200° C einführbar sind, ganz bevorzugt wobei der Einspritzvorgang ein Co-Einspritzvorgang ist, in welchem das PETG bei einer niedrigen Temperatur als die zwei Schichten auf beiden Seiten der Vorform injiziert werden kann; Insbesondere wobei die lebenden Organismen in dem PETG der Spezies *Bacillus Subtilis* angehören.

9. Verfahren nach einem der Ansprüche 1 oder 2 bis 8, **dadurch gekennzeichnet, dass** das tertiäre Material (C), welches ein wärmeempfindliches Material ist, über einen Weg kalt zugeführt wird, der nicht wärmebelastet (69) ist, insbesondere unter virtuellen normalen Bedingungen einer Raumtemperatur und eines Umgebungsdrucks; und/oder
insbesondere, wenn abhängig nach einem der Ansprüche 7 oder 8, dadurch, dass unter dem Betriebstemperaturbereich gearbeitet wird, dessen untere Grenze bei im Wesentlichen 100° C unter Druckbedingungen p ≥ 1 atm eingestellt ist, insbesondere unter normalen Druckbedingungen von etwa 1 atm, vorzugsweise bei einer Temperatur von bis zu 30° bis 40° C, und/oder
dadurch, dass das tertiäre Material (C) mit dem sekundären Material (B) in eine zentrale Region (52) mitgenommen wird, die in der Mitte der primären Doppelbasisschicht (11) liegt, und zwar unter Bildung einer inneren Barriereschicht (12), welche aus dem sekundären Material (B) und dem tertiären Material (C) als Aktivator- oder Initiatormaterial zusammengesetzt ist, welches hier eingebaut wird.

10. Verfahren nach einem der vorhergehenden Ansprüche, **dadurch gekennzeichnet, dass** die Barrierematerialien (B & C) gleichmäßig mit sehr geringen Materialmengen verwendet werden, insbesondere das tertiäre Material (C); und/oder dadurch, dass eine vorbestimmte Menge von Zusatzstoffen vorher zu wenigstens einem der Materialien mit neutralisierendem Effekt auf unerwünschte Einflüsse zugeführt wird, insbesondere gegenüber eine äußere Strahlung und/oder Substanzen, insbesondere eine elektromagnetische Strahlung, insbesondere eine UV-Strahlung, eine IR-Strahlung, eine γ-Strahlung und/oder sichtbares Licht oder gegenüber Reaktionsmittel, welche einen nachteiligen Einfluss auf einem zu konservierenden Produkt haben.

11. Spritzgussvorrichtung zum Herstellen von hohlen Gegenständen, insbesondere mehrschichtigen Kunststoffvorformlingen (51), mit einer Spritzgussform (61), mit einer Heißkanalplatte (65) und einem paar von Einspritzdüsen (86), welche mit einem im Wesentlichen zentral angeordneten Hauptzuführkanal (79) versehen sind, an dessen freiem Ende ein Stranggussverbindungskanal (62) vorgesehen ist, welcher mit Hilfe einer Blockierstange (71) verschließbar ist, welche darin bewegbar ist und welche durch einen profilierten inneren Teil hin und her bewegbar ist, welcher in einem Träger aufgenommen ist, um welchen sich ein Heizelement herum erstreckt, innerhalb welchem wenigstens ein primärer Kanal (67) für die warme Zuführung eines Kunststoff-Basismaterials (A) zu dem Gießkanal (62) verläuft, wobei ein separater sekundärer Kanal (68) für die separate Zuführung des sekundären Trägermaterials (B) zu der Stranggussverbindung (62) vorgesehen ist, der gemeinsam genutzt wird, **dadurch gekennzeichnet, dass** das sekundäre Material (B) zuerst warm und separat von dem primären Material (A) zuführbar ist, und zwar über den sekundären Kanal (68), und nachfolgend stromabwärts davon, kalt von einem weiteren Strangguss-Bindungskanal (62) kalt zuführbar ist und dadurch, dass ein tertiäre Zuführkanal (69) ferner für eine separate Zuführung einer vorbestimmten Menge eines tertiären Materials als Aktivatormaterial (C) unabhängig von dem sekundären Material (B) vorgesehen ist, der außerhalb der Heißkanalplatte (65) liegt und der sich von dieser nach außen zu dem sekundären Zuführkanal (68) erstreckt, wobei der tertiäre Zuführkanal (69) in den weiten Strangguss-Verbindungskanal (72) läuft, welcher in einem vorbestimmten Abstand (69) zu der gemeinsamen Strangguss-Verbindung (62), stromaufwärts davon, angeordnet ist, und zwar unter Temperatur- bzw. Druckbedingungen, welche deutlich niedriger sind als die primäre und sekundäre Injektionstemperatur und/oder Druck p, so dass das tertiäre Material (C) zusammeln mit den sekundären Material (B) von dem weiteren Strangguss-Verbindungskanal (72) zu den gemeinsamen Stranggusskanal (62) mitgenommen wird, um gemeinsam in die Spritzgussform (60) in einer gesteuerten mit dem tertiären Material (C), welches über den Umgehungskanal (69) zuführbar ist, einspritzbar zu sein, und zwar über eine für diesen Zweck geeignet vorgesehene Steuereinrichtung, die aus der kooperierenden Blockierstange (71) besteht, welche abwechselnd zwischen einer gegenwärtigen primären Position für die primäre Zuführung und eine weitere Position für die Zuführung des zusammengesetzten Barrierematerials (B, C) bewegbar ist;
wobei eine sekundäre Zuführeinheit (92) vorgesehen ist, welche von der heißen primären Zuführeinheit (91) unabhängig ist und welche außerhalb der primären Zuführeinheit (91) angeordnet ist, durch welche sekundäres Material (B) von einer unabhängigen Stromversorgungseinheit (99) über einen sekundären Zuführkanal (68) zugeführt werden kann, der dafür vorgesehen ist, und dadurch, dass ein hierin verlaufender tertiärer Zuführkanal (69) vorgesehen ist, welcher außerhalb der sekundären Zuführeinheit (92) angeordnet ist, und zwar wenigstens in deren wärmebelasteten Teil, der stromaufwärts der weiteren Strangguss-Verbindung (62) angeordnet ist, durch welchen das tertiäre Material (C) von einer unabhängigen Leistung-Versorgungseinheit (93) über den weiteren unabhängigen, dafür vorgesehenen Zuführkanal (69) kalt zugeführt werden kann.

12. Vorrichtung nach dem vorhergehenden Anspruch, insbesondere zum Herstellen einer Vorform in einer Spritzgussform, **dadurch gekennzeichnet, dass** der tertiäre Zuführkanal (69) mit dem sekundären Zuführkanal (68) in dem weiteren Strangguss-Verbindungskanal (72) verbunden ist, welcher wiederum mit dem primären Zuführkanal (67) in einer gemeinsamen Injektionsdüse (86) verbunden ist, welche für die Herstellung der Vorform (51) bzw. des Behälters (50) direkt in die Form (62) läuft, in deren Injektionsstelle (62).

13. Vorrichtung nach dem vorhergehenden Anspruch, **dadurch gekennzeichnet, dass** die Verbindung (77) mit der Strangguss-Verbindung (62) in einem relativ kurzen Abstand (79) in Bezug auf den Eintrag des primären Zuführkanals (11) in der Spritzgussdüse (86) versehen ist.

## Revendications

1. Procédé de fabrication d'objets creux, en particulier des préformes multicouches en matière plastique (51), qui sont réalisées dans un espace de moulage creux (60) prévu à cet effet, à partir d'au moins deux matériaux comprenant un matériau de base primaire (A) et un matériau secondaire (B) comme matériau de support, dans lequel le matériau de base (A), et éventuellement le matériau secondaire (B), est formé par une matière plastique, et dans lequel tant les matériaux primaire de base que secondaire de support (A, resp. B) sont amenés dans l'espace de moule creux (60) par des canaux d'alimentation primaire et secondaire (67, 68) dans un moule d'injection (61) et une plaque à canaux chauffants (65), chacun d'entre eux (67, 68) passant par des éléments de chauffage adaptés et conduisant leurs matières primaire de base et secondaire de support respectives (A, resp. B) à une connexion d'extrusion commune (62) à travers laquelle les matériaux primaire de base et secondaire de support (A, B) sont injectés séparément dans l'espace de moulage creux (60), d'abord avec le matériau primaire de base (A) qui est injecté sous une température T et une pression p relativement élevées, **caractérisé en ce qu'**une quantité prédéterminée d'un matériau tertiaire (C) est en outre amenée comme matière d'activation, indépendamment des deux autres matériaux (A, B), extérieurement à et en dehors de ladite plaque à canaux chauffants (65), audit canal d'alimentation secondaire (68) dans lequel aboutit (66) un conduit d'alimentation tertiaire (69) à une distance prédéterminée de ladite connexion d'extrusion commune (62), en amont de celle-ci, dans des conditions de température et/ou de pression qui sont nettement inférieures à celles de ladite température T et/ou de pression p d'injection primaire et secondaire, de sorte que ledit matériau tertiaire (C) est entraîné avec ledit matériau secondaire (B), afin d'être injecté en même temps dans l'espace creux (60) d'une manière contrôlée, avec formation d'une couche intermédiaire composite formant barrière (12); et **en ce que** ledit matériau (C) est amené au moyen d'un insert (5) ou torpille qui est placée de manière appropriée à cet effet dans le canal d'alimentation secondaire à la périphérie du système à canaux chauffés (65) juste avant le trou de coulée (62), dans lequel une quantité de matériau primaire (A) est d'abord injectée dans ledit moule de formage (60) par l'intermédiaire dudit raccord d'extrusion (62), après quoi ledit matériau secondaire (B) est alimenté individuellement par rapport audit matériau primaire (A) par l'intermédiaire du canal secondaire (68), dans lequel une tige de verrouillage (71) coopérant est déplacée de sa position initiale actuelle dans laquelle le canal secondaire (68) est bloqué, à une autre position dans laquelle le canal secondaire (68) est ouvert et permet un passage cyclique du matériau de barrière (B, C), par une connexion d'extrusion supplémentaire (72) à travers laquelle les matériaux secondaire (B) et tertiaire (C), qui est incorporé par l'intermédiaire du canal de dérivation (69), sont injectés, dans le but d'être injecté en même temps dans l'espace creux du moule (60) d'une manière contrôlée.

2. Procédé selon la revendication 1, dans lequel une quantité prédéterminée de matière plastique primaire, resp. secondaire (A, B) est injectée dans l'espace de moulage creux (60) sous une pression p et une température T relativement élevées, **caractérisé en ce que** le matériau de base primaire (A) est injecté sans interruption, resp. en continu, tandis que le matériau de barrière (B et C) est amené audit matériau primaire (A) pendant la phase d'injection à travers un point de connexion (77), et est ainsi entraîné sur une courte distance (79) vers ledit espace de moulage (60).

3. Procédé selon l'une des deux revendications précédentes, pour la fabrication d'une préforme (51) dans un moule d'injection, dans lequel la préforme constitue une forme en creux étant destinée à la fabrication de récipients (50), **caractérisé en ce que** ladite préforme est réalisée avec une structure multicouches comprenant au moins une première couche de base double, qui se compose d'un matériau primaire (A) consistant en un polymère, une double couche de base primaire (11) étant réalisée sous forme d'une matrice d'enrobage, qui constitue la surface extérieure de la préforme délimitant celle-ci, et qui est composée en outre d'une couche intermédiaire secondaire (12), qui est incorporée dans ladite double couche de base primaire (11) comprenant un matériau secondaire (B), dans lequel ledit matériau secondaire (B) est constitué d'un matériau de support dans lequel un matériau tertiaire (C) est incorporé en tant que matériau initiateur ou resp. activateur formant ainsi une couche composite intermédiaire interne (12) formant barrière.

4. Procédé selon la revendication précédente, **caractérisé en ce que** ledit matériau primaire (A) est sélectionné à partir d'un polymère plastique; et/ou
**en ce que** ledit matériau de support (B) est sélectionné à partir d'un polymère, en particulier un polymère synthétique, plus particulièrement un biopolymère; et/ou en ce que ledit matériau tertiaire activateur (C) est sélectionné à partir d'un fluide, en particulier un liquide, notamment un liquide visqueux, plus particulièrement un fluide à base d'huile; ou un liquide contenant de l'eau, respectivement un liquide aquifère; ou un acrylate, le cas échéant à base d'huile ou d'eau; ou **en ce que** ledit fluide est constitué par un gaz.

5. Procédé selon l'une des revendications 3 ou 4, **caractérisé en ce que** ladite couche secondaire (12) est sélectionnée à partir d'une phase dite intermédiaire, telle que des pâtes, des adhésifs et d'autres substances qui ont une phase liquide dans des conditions normales de pression et de température, et qui peut éventuellement se transformer en une phase solide, notamment par durcissement; en particulier dans lequel au moins une couche intermédiaire supplémentaire (13, 14) est pourvue de la double couche primaire adjacente (11) au moins sur un côté de contact de ladite couche secondaire (12), qui (13, 14) est composée d'un matériau quaternaire (D).

6. Procédé selon l'une des revendications précédentes, **caractérisé en ce que** ledit matériau tertiaire activateur (C) est sélectionné à partir desdits photo-initiateurs (95); et/ou en ce qu'une quantité prédéterminée d'additifs (41, 43), notamment des colorants, est ajoutée à au moins l'un desdits matériaux (A, B, C, D) ou aux couches (11, 12, 13, 14); en particulier dans lequel lesdits additifs ont une action neutralisante sur le rayonnement et/ou des substances externes, en particulier le rayonnement électromagnétique, plus particulièrement le rayonnement UV, le rayonnement infrarouge, le rayonnement γ et/ou la lumière visible, le cas échéant l'induction, et/ou **en ce que** lesdits additifs ont une action neutralisante sur les agents réactifs ayant un effet néfaste sur un produit à conserver (58), et/ou **en ce que** lesdits additifs (41) ont une action neutralisante sur la formation indésirable de gaz provenant de la dégradation dudit produit (58), et/ou **en ce que** lesdits additifs ont une action neutralisante sur les déchets ou matériaux dégradés provenant de la préforme elle-même.

7. Procédé selon l'une des revendications 3 à 6, **caractérisé en ce que** la couche intermédiaire formant barrière (12) est réalisée à partir de ce qu'on appelle un bio-agrégat de polymère qui est constitué par des cellules et/ou des produits cellulaires en tant que matériau activateur qui est incorporé dans ledit polymère secondaire comme matériau de support; en particulier dans lequel lesdites cellules consistent **en ce qu'**on appelle des kystes et/ou appartiennent à la phase de stades inactifs ou dormants; ou à partir de levures, de procaryotes, notamment des bactéries, et/ou des eucaryotes, notamment du type protistes, des champignons et/ou des plantes; resp.
**en ce que** lesdits produits cellulaires consistent en métabolites, étant des molécules qui sont synthétisables par des organismes selon une voie biochimique.

8. Procédé selon la revendication précédente, **caractérisé en ce que** lesdits organismes vivants sont incorporés dans le polymère dans une phase liquide pendant le moulage par injection à une température supérieure à 260°C; ou quand il est destiné à la fabrication de récipients réalisés à partir d'un polymère comprenant des organismes vivants, constitués d'au moins une couche (2), **en ce que** cette couche est constituée de polyéthylène téréphtalate glycol PETG comprenant des organismes vivants, dans lequel ledit polymère est du PETG possédant une température de fusion inférieure au PET standard, en particulier dans lequel ladite préforme est constituée d'au moins trois couches, dans laquelle la couche de PETG est une couche intermédiaire qui est comprise entre les deux autres couches, plus particulièrement dans lequel lesdits organismes vivants peuvent être introduits dans le PETG dans une phase liquide pendant le procédé de moulage par injection à une température supérieure à 200°C, encore plus particulièrement dans lequel le moulage par injection est un procédé de moulage par co-injection, dans lequel le PETG peut être injecté à une température plus froide que les deux couches des côtés intérieur et extérieur de la préforme; en particulier dans lequel lesdits organismes vivants dans le PETG appartiennent à *l'espèce Bacillus subtilis.*

9. Procédé selon l'une des revendications 1 ou 2 à 8, **caractérisé en ce que** le matériau tertiaire (C), qui est un matériau sensible à la chaleur, est amené à froid par l'intermédiaire d'une voie non chargée thermiquement (69), notamment dans des conditions pratiquement normales de température ambiante et de pression atmosphérique; et/ou en particulier lorsqu'elle dépend de l'une des revendications 7 ou 8, **en ce qu'**on opère dans l'intervalle de température de fonctionnement, dont la limite inférieure est fixée à pratiquement 100°C dans des conditions de pression p ≥ 1 bar, notamment dans des conditions normales de pression d'environ 1 atm, de préférence à une température allant jusqu'à un intervalle compris entre 30°C et 40°C; et/ou
**en ce que** le matériau tertiaire (C) est entraîné avec le matériau secondaire (B) dans une région centrale (52) qui est comprise au milieu de ladite double couche de base primaire (11), avec la formation d'une couche intérieure formant barrière (12) qui est composée de matériau secondaire (B) et de matériau tertiaire (C) comme matériau activateur ou initiateur qui y est incorporé.

10. Procédé selon l'une des revendications précédentes, **caractérisé en ce que** les matériaux de barrière (B et C) sont appliquées de manière uniforme avec de très faibles quantités de matière, plus particulièrement la matière tertiaire (C); et/ou **en ce qu'**une quantité prédéterminée d'additifs est préalablement introduite dans au moins l'une desdites *matières* avec un effet neutralisant sur les influences indésirables, en particulier sur le rayonnement et/ou des substances externes, notamment le rayonnement électromagnétique, plus particulièrement le rayonnement UV, le rayonnement infrarouge, le rayonnement γ et/ou la lumière visible, ou sur des agents réactifs qui ont un effet défavorable sur un produit à conserver.

11. Dispositif de moulage par injection pour la fabrication d'objets creux, en particulier des préformes en plastique multicouches (51), comprenant un moule à injection (61), avec une plaque à canaux chauffants (65) et une paire de buses d'injection (86), qui sont chacun munis d'un conduit d'alimentation principal (79) disposé sensiblement au centre à l'extrémité libre duquel un trou de coulée (62) est prévu, qui peut être fermé au moyen d'une tige de verrouillage (71), qui y est mobile et qui peut être déplacée à va-et-vient à travers une pièce intérieure profilée qui est reçue dans un support autour duquel un élément chauffant s'étend à l'intérieur duquel au moins un canal primaire (67) passe pour la fourniture à chaud d'un matériau de base en plastique (A) à la connexion d'extrusion (62), dans lequel un canal secondaire distinct (68) est prévu pour l'alimentation séparée du matériau de support secondaire (B) à la connexion d'extrusion (62) qui est commune, **caractérisé en ce que** ledit matériau secondaire (B) est délivrable d'abord à chaud et séparément du matériau primaire (A), par le canal secondaire (68), et ensuite délivrable à froid en aval de celui-ci, à partir d'une carotte de connexion d'extrusion supplémentaire (72), et **en ce que**, en outre, un conduit d'alimentation tertiaire (69) est prévu pour l'apport séparé d'une quantité prédéterminée de matériau tertiaire comme matériau activateur (C) indépendamment du matériau secondaire (B), qui est extérieur à la plaque à canaux chauffants (65) et qui s'étend à l'extérieur de celui-ci vers ladite conduite d'alimentation secondaire (68), dans lequel le conduit d'alimentation tertiaire (69) aboutit dans la connexion d'extrusion supplémentaire (72) qui est disposée à une distance prédéterminée (79) du raccord d'extrusion commun (62), en amont de celui-ci, dans des conditions de température resp. de pression qui sont significativement inférieures à ladite température T et/ou de la pression p d'injection primaire et secondaire, de sorte que le matériau tertiaire (C) peut être alimenté de concert avec le matériau secondaire (B) depuis la connexion d'extrusion supplémentaire (72) vers la coulée d'extrusion commune (62), afin d'être injectable conjointement dans l'espace de moulage creux (60) dans un mode contrôlé avec le matériau tertiaire (C), qui est délivrable par le canal dérivé (69), par l'intermédiaire d'un moyen de commande approprié prévu à cet effet, comprenant la tige de verrouillage coopérant (71), qui peut être déplacée alternativement entre une position initiale actuelle pour l'alimentation primaire, et une autre position pour l'amenée du matériau composite formant barrière (B, C), dans lequel il est prévu une unité d'alimentation secondaire (92), qui est indépendante de l'unité d'alimentation primaire à chaud (91) et qui est agencée extérieurement à ladite unité d'alimentation primaire (91) par lequel du matériau secondaire (B) peut être alimenté à partir d'une unité d'alimentation indépendante (99) par l'intermédiaire d'un conduit d'alimentation secondaire (68) prévu à cet effet et **en ce qu'**il est prévu un conduit d'alimentation tertiaire (69) passant dans celui-ci, qui est disposé à l'extérieur de ladite unité d'alimentation secondaire (92) au moins dans la partie chargée thermiquement de celui-ci qui est située en amont dudit raccord d'extrusion supplémentaire (72), par lequel le matériau tertiaire (C) peut être amené à froid à partir d'une unité d'alimentation énergétique indépendante (93) par l'intermédiaire du conduit d'alimentation indépendant supplémentaire (69) prévu à cet effet.

12. Dispositif selon la revendication précédente, en particulier pour la fabrication d'une préforme dans un moule d'injection, **caractérisé en ce que** le conduit d'alimentation tertiaire (69) est relié à la conduite d'alimentation secondaire (68) dans le raccordement d'extrusion supplémentaire (72), qui à son tour, est relié ensuite au conduit d'alimentation primaire (67) dans une buse d'injection commune (86), qui va directement dans le moule (60) pour la réalisation de la préforme (51), resp. du récipient (50), dans le point d'injection (62) de celui-ci.

13. Dispositif selon la revendication précédente, **caractérisé en ce que** ladite connexion (77) est pourvue du raccordement d'extrusion (62) à une distance relativement courte (79) par rapport à l'entrée du canal d'alimentation primaire (11) dans ladite buse de moulage par injection (86).
